# EUROPEAN PATENT SPECIFICATION

(11) **EP 2 812 025 B1**
(45) Date of publication and mention of the grant of the patent: **19.07.2017**
(21) Application number: 13702074.9
(22) Date of filing: 04.02.2013
(51) Int. Cl.: A61K 39/35

(54) **METHOD OF PREVENTING ALLERGIES**
VERFAHREN ZUR VORBEUGUNG VON ALLERGIEN
PROCÉDÉ DE PRÉVENTION DES ALLERGIES

(30) Priority: 06.02.2012 US 201261595577 P; 13.02.2012 EP 12305153
(43) Date of publication of application: 17.12.2014
(73) Proprietor: DBV Technologies, 92120 Montrouge (FR)
(72) Inventor: BENHAMOU, Pierre-Henri, F-75116 Paris (FR); DUPONT, Christophe, F-92140 Clamart (FR); MONDOULET, Lucie, 92320 Chatillon (FR)
(74) Representative: Becker, Philippe
(86) International application number: PCT/EP2013/052163
(87) International publication number: WO 2013/117519

(56) References cited:
- WO-A1-2009/071599
- WO-A2-2009/080934
- DUPONT CHRISTOPHE ET AL: "Cow's milk epicutaneous immunotherapy in children: a pilot trial of safety, acceptability, and impact on allergic reactivity.", THE JOURNAL OF ALLERGY AND CLINICAL IMMUNOLOGY MAY 2010 LNKD- PUBMED:20451043, vol. 125, no. 5, May 2010 (2010-05), pages 1165-1167, XP002676152, ISSN: 1097-6825
- DUPONT C ET AL: "Epicutaneous Immunotherapy In Severe Cow Milk Allergy: A Double Blind Pilot Trial", JOURNAL OF ALLERGY AND CLINICAL IMMUNOLOGY, vol. 123, no. 2, Suppl. S, February 2009 (2009-02), page S183, XP002676153, & 65TH ANNUAL MEETING OF THE AMERICAN-ACADEMY-OF-ALLERGY-ASTHMA-AND-IMM UNOLOGY; WASHINGTON, DC, USA; MARCH 13 -17, 2009 ISSN: 0091-6749
- MONDOULET L ET AL: "In Mice Sensitized to Milk, Epicutaneous Immunotherapy Prevents Further Sensitization to Peanut or House Dust Mite", JOURNAL OF ALLERGY AND CLINICAL IMMUNOLOGY, vol. 129, no. 2, Suppl. S, February 2012 (2012-02), page AB113, XP002676154, & ANNUAL MEETING OF THE AMERICAN-ACADEMY-OF-ALLERGY-ASTHMA-AND-IMM UNOL OGY (AAAAI); ORLANDO, FL, USA; MARCH 02 -06, 2012
- L. MONDOULET ET AL: "Epicutaneous immunotherapy on intact skin using a new delivery system in a murine model of allergy", CLINICAL & EXPERIMENTAL ALLERGY, vol. 40, no. 4, 1 April 2010 (2010-04-01), pages 659-667, XP055003086, ISSN: 0954-7894, DOI: 10.1111/j.1365-2222.2009.03430.x
- DIOSZEGHY VINCENT ET AL: "Epicutaneous Immunotherapy Results in Rapid Allergen Uptake by Dendritic Cells through Intact Skin and Downregulates the Allergen-Specific Response in Sensitized Mice", JOURNAL OF IMMUNOLOGY, vol. 186, no. 10, May 2011 (2011-05), pages 5629-5637, XP002676155, ISSN: 0022-1767
- PRADO N ET AL: "Bystander suppression to unrelated allergen sensitization through intranasal administration of tolerogenic exosomes in mouse", MOLECULAR IMMUNOLOGY, PERGAMON, GB, vol. 47, no. 11-12, 1 July 2010 (2010-07-01), pages 2148-2151, XP027072113, ISSN: 0161-5890, DOI: 10.1016/J.MOLIMM.2010.04.014 [retrieved on 2010-05-15]
- DIOSZEGHY VINCENT ET AL: "Epicutaneous immunotherapy results in rapid allergen uptake by dendritic cells through intact skin and downregulates the allergen-specific response in sensitized mice.", JOURNAL OF IMMUNOLOGY (BALTIMORE, MD. : 1950) 15 MAY 2011, vol. 186, no. 10, 15 May 2011 (2011-05-15) , pages 5629-5637, ISSN: 1550-6606

## Description

### FIELD OF THE INVENTION

The present invention discloses compositions and methods for preventing allergy in a subject. Particularly, the present invention relates to epicutaneous prevention of allergies in children. The invention also discloses compositions for use in such methods, as well as their manufacture, and their use to cause protection against any allergy, particularly food or respiratory allergies.

### BACKGROUND OF THE INVENTION

Allergy occupies an increasingly large place in daily medical practice. It is a worldwide public health phenomenon classified as the fourth worldwide scourge by the WHO. In France, one Frenchman out of three is allergic and both respiratory and food allergy affects an increasing portion of the population of adults and children.

Desensitization (or immunotherapy) is the technique according to which by administering minimal amounts of allergens it is possible to more or less suppress the allergic reaction to these allergens. By progressively administering the allergen, in constant or varying doses, the subject becomes tolerant to the allergen. Immunotherapy has proved to be effective in patients affected with severe IgE-dependent allergy, sensitized to a restricted number of allergens. Desensitization can reduce the symptoms related to short term allergic reaction as well as the progression of the symptomatology towards more severe clinical signs, such as for example the transformation of allergic rhinitis into asthma (Tij *et al*., 2004). Since the beginning of the year 2000, it has been recognized by the WHO as the only method for basically treating allergy.

Although the mechanisms of action of immunotherapy are not yet well-known, they appear to involve (i) an increase in the IgGs and, in particular, in IgG4 fractions, these antibodies blocking *in vitro* the biological effects of IgEs; (ii) a modification of the TH1 and TH2 immunological response, promoting a more balanced TH1/TH2 response; and/or (iii) a production of T cells producing interleukin 10 (IL-10), which display anti-allergic properties against mastocytes, certain T lymphocytes and eosinophils and also promote the production of IgG4.

Immunotherapy may be accomplished by various protocols or routes, including subcutaneous, sublingual, nasal or epicutaneous. Subcutaneous Immunotherapy is widely used. Although costly and requiring the intervention of a specialist physician at each injection, it is today still considered as the standard desensitization route in children (Pajno *et al*., 2005). However it is not without risks, since the frequency of deaths is estimated to be 1 for 2.5 million injections with an average of 3.4 deaths per year. Furthermore, potentially anaphylactic allergens with a high risk of reaction, such as tree nuts and most food allergens, are not used for subcutaneous desensitization treatment.

Sublingual immunotherapy is considered by the WHO as a satisfactory alternative to subcutaneous immunotherapy. Much better tolerated by the patients, sublingual immunotherapy is considered by many authors to provide a better cost-effectiveness ratio than the other routes of administration, since it does not require the intervention of a physician and may be self-administered by the patients (Pajno *et al*., 2005). Also, adverse reactions are rare during treatments via a sublingual route, estimated to be from 0.1 to 0.2 reactions for 1,000 administered doses. Such reactions are mainly minor, affecting the buccal cavity or the sublingual area or the gastro-intestinal sphere (La Rosa *et al*., 1999).

In terms of effectiveness, however, the sublingual route is considered as less effective than the subcutaneous route: it requires the use of a larger amount of allergen and cannot be authorized with all allergens. This is so even though tests via this route for desensitization to groundnuts and to cow milk are in progress. The safety of the use of this technique with other food allergens remains to be evaluated.

Intra-nasal immunotherapy has proved to be effective in 17 controlled studies out of 18. This is therefore an effective and safe route for treating at least allergic rhinitis. However, it is generally poorly tolerated by patients, which increasingly limits its clinical use. In a study comparing different desensitization routes, treatments via nasal route were prematurely interrupted in approximately 50% of the cases before one year, while early cessation occurred in less than 10% of the children treated via a subcutaneous or sublingual route (Pajno *et al*., 2005). Prado et al., 2010 Mol. Immunol., vol. 47, no. 11-12, pages 2148-2151 concerns intranasal application of bioactive nanovesicles called exosomes specific to particular olive allergens, and their use in preventing the sensitization to allergens from birch pollen. Epicutaneous immunotherapy is based on skin application. This method typically comprises the repeated application of an allergen known to cause an existing allergy on the skin of a subject, generally leading to diffusion of the allergen in the surface layers of the skin. Experiments conducted by the inventors have shown that the type of immune response generated by epicutaneous immunotherapy may be controlled by the treatment conditions. For example, commonly assigned international application WO2009/080934 discloses that a potent desensitization to an allergen may be obtained by epicutaneous therapy using an allergen, without adjuvant, applied on an intact area of the skin. Similarly, commonly assigned international application WO2009/071599 discloses that a strong desensitization to groundnut may be obtained by epicutaneous immunotherapy. Dioszeghy et al., 2011 J.Immunol., vol. 186, no.10, pages 5629-4637, concerns mechanisms of epicutaneous immunotherapy through intact skin in mice sensitized with ovalbumin.In the above experiments and publications, the allergen-specific effects of epicutaneous immunotherapy were investigated by the inventors. Continuing that research, the inventors have now surprisingly found that, depending on the treatment conditions, epicutaneous immunotherapy may be employed as a prophylactic treatment to prevent development of allergies in allergic patients. This unexpected result therefore allows the design of a prophylactic treatment to prevent allergies in subjects.

### SUMMARY OF THE INVENTION

The present application provides novel methods for preventing allergies in mammalian subjects by prophylactic epicutaneous administration of an allergen. The invention discloses a method for preventing allergies in a child, wherein the method comprises the epicutaneous administration of an allergen to said child, the allergen being administered to the child upon detection of an allergic reaction to the same allergen or a different allergen.

The invention also discloses a method for preventing allergy in a child comprising epicutaneously administering to a child at least one allergen, wherein the child is under 6 years old and has a detected allergic reaction to at least one allergen. The child is preferably an infant or a toddler.

The invention also discloses a method for preventing allergy in a child, wherein the child is allergic to milk or house dust mite and is under 6 years old, and wherein the method comprises epicutaneously administering to said child at least one milk or house dust mite protein. As will be disclosed in the application, such epicutaneous administration prevents the onset or development of allergy to other allergens in said child.

The invention also discloses a method for preventing allergy in a child, the method comprising detecting or verifying in a child the occurrence of a first allergic reaction and, upon detection or verification of said first allergic reaction, epicutaneously administering to said child at least one allergen responsible for said first allergic reaction, said epicutaneous administration protecting the child from development of at least one further allergy to another allergen.

The invention also discloses a method for protecting a child from development of multiple allergies, the method comprising detecting or verifying in a child the occurrence of an allergic reaction and, upon detection or verification of said allergic reaction, epicutaneously administering to said child at least one allergen responsible for said allergic reaction, said epicutaneous administration inducing an immune response in said child protecting the child from development of multiple allergies.

The invention also discloses a composition for use in preventing allergies in a subject, wherein the composition comprises an allergen and is applied epicutaneously to an allergic child. More preferably, the child is under 6 years old, more preferably the child is an infant or a toddler. Even more preferably, the child is allergic to milk or house dust mite. Most preferably, the child is treated upon detection of his first allergy.

As it is disclosed herein, the invention may be used to prevent development of further allergies in the treated subjects, including to food, respiratory or contact allergies. It is particularly well suited to the treatment of infants or toddlers who exhibit a first allergic event to a first allergen, to desensitize the child to the first allergen, and to prevent the development of further allergies to other, different allergens.

More specifically, the invention relates to a composition as defined in the claims.

In a preferred embodiment, the invention relates to a composition comprising a milk allergen for use in treating a child under 6 years old having a detected allergy to milk, to prevent said child from developing a further allergy to a different food allergen, wherein the composition is repeatedly administered epicutaneously to said child using a non-invasive occlusive patch device, said administration protecting the child from development of at least one further allergy to the different food allergen. The child is preferably an infant or a toddler. The detected allergy to milk is preferably the first detected allergy in said child and the allergen is administered preferably upon detection or verification of said first allergic reaction. The allergen is preferably applied on an intact area of the skin and/or in the absence of adjuvant. Also, the allergen is preferably applied repeatedly during a period of 3 to 36 months.

In another preferred embodiment, the composition of the invention is for protecting said child from development of multiple allergies.

In another preferred embodiment, the composition of the invention is for protecting said child from development of an allergy to peanut.

### LEGEND TO THE FIGURES

Figure 1: study design. BALB/c mice were sensitized to milk and divided into 2 groups in which one was treated (EPIT) and the other was untreated (Sham). The treatment was conducted for 8 weeks. After this period, all mice received peanut with the same protocol ordinarily used to sensitize mice to peanut protein extract (PPE). Mice were then exposed to peanuts for 10 days. Esophagus inflammation was evaluated by the measurement of eosinophil infiltration into esophagus, as determined by histology. Serological responses and cellular responses were also investigated.
Figures 2: Specific IgE (Figure 2a) and IgG2 (Figure 2b) responses to milk after sensitization at day 43 (D43), during EPIT at day 78 (D78), and at the end of treatment, day 99 and day 127 (D99 and D127). Results are expressed as mean ± SD. ** p<0.01.
Figure 3: Specific IgE (Figure 3a) and IgG2 (Figure 3b) responses to PPE after EPIT but before being exposed to PPE (D99) and after being exposed to PPE (D127). Results are expressed as mean ± SD. * p<0.05, *** p<0.001.
Figure 4: Cellular responses of in vitro reactivated splenocytes by milk (CMP) or PPE as measured by IL-4 (Figure 4a) and IL-5 (Figure 4b). Results are expressed as mean ± SD. * p<0.05.
Figure 5: Induction of Foxp3+CD25+CD4+ T cells (Treg) from in vitro reactivated splenocytes. At the end of experiments, splenocytes were harvested and reactivated in vitro for 3 days in the presence of milk (left part of the graph) and either PPE (right part of the graph). The percentage of Treg per total CD4+ T cell population was expressed as mean±SD. * p<0.05, ** p<0.01.
Figure 6: Eosinophil infiltration into esophagus evaluated by histological analyses. Results are expressed as mean ± SD. ** p<0.01.

### DETAILED DESCRIPTION OF THE INVENTION

The invention provides a novel prophylactic treatment of allergies. It is particularly well suited to treat children exhibiting an allergic reaction to an allergen, to protect them against development of further allergies to other, different allergens. The present application discloses that it is particularly efficient to treat young children who show an allergic reaction to milk or house dust mite, thereby protecting the children from developing further allergies to still other allergens. Indeed, young children, such as infants or toddlers, who develop an allergic reaction to a first allergen, are very susceptible to become allergic to other substances. The present invention allows the treatment of such allergic subjects and reduces the occurrence of further allergies.

The present disclosure will be best understood by reference to the following definitions:

### Definitions

"Epicutaneous administration" refers to the application of a substance (e.g., an allergen) on the skin of the subject under conditions allowing a contact with the surface of the skin. Skin application is preferably performed without any skin perforation or pre-treatment. Skin application is preferably maintained in conditions allowing penetration of the allergen in the superficial layer(s) of the skin and/or and for a period of time sufficient to allow contact of the allergen with immune cells. Epicutaneous administration is preferably performed with a skin device, such as a patch.

Within the context of this invention, the term "preventing" allergies is meant to include protecting the subject from development of further allergies, preventing appearance of a sensitivity to further allergens, delaying appearance or occurrence of such sensitivity or allergy to further allergens, or inhibiting or reducing the magnitude of any further allergic reaction.

Within the context of the present invention, the term "intact skin" indicates that the integrity of the stratum corneum layer should be substantially maintained. For the performance of the invention, it is indeed most preferred to apply antigens on intact (i.e., non-pre-treated) skin, e.g., on a surface or portion of the skin where the integrity of the stratum corneum is essentially maintained. Maintaining the integrity of the stratum corneum and natural activation state of keratinocytes and Langerhans cells, which are located below the stratum corneum, is important to avoid a biased Th2 immune response. By maintaining this integrity, the response obtained is highly oriented in the sense of tolerance. Accordingly, although gentle cleaning of the skin surface may be performed at the site of application, e.g., hydration, water cleaning, or very gentle single stripping, to remove e.g., corneocytes, the skin should not be pre-treated, thus maintaining substantial integrity of the stratum corneum. In particular, tape-stripping or strong abrasion of the skin should not be performed, since such pre-treatments disrupt, or remove all or part of the stratum corneum and cause stimulation of keratinocytes. Similarly, perforation of the stratum corneum should be avoided.

The term "allergen" refers to an immunologic molecule involved in an allergic reaction. The allergen may be of various nature, such as a lipid, protein, peptide, polypeptide, metal, plastic, etc. In a particular embodiment, the allergen is a protein, polypeptide and/or peptide. The allergen may be in a natural state, or produced artificially (e.g., by recombinant and/or enzymatic techniques for instance). The allergen may be structurally altered or modified to improve its stability, immunogenicity, etc. The allergen may be pure or in admixture with other constituents. The allergen may be a mixture of several molecules (e.g., an extract). As will be discussed further below, the allergen may be used in different states, such as liquid or dry.

### Methods for preventing allergies

The present invention discloses compositions and methods for preventive treatment of allergies. In particular, the inventors have made the unexpected discovery that epicutaneous administration of an allergen can induce or stimulate a non allergen-specific immune response in a subject, thereby protecting the subject from development of further allergies to other, different allergens. By applying epicutaneous immunotherapy in subjects having an allergic background, it is possible not only to desensitize an existing allergy but also to prevent the development of further allergies. As discussed in detail below, the inventors' in vivo experiments demonstrate that the epicutaneous application of milk proteins to mammals allergic to milk desensitizes against milk and also protects against challenge by other allergens, including peanut and dust mite. These results are particularly surprising and remarkable since using a food allergen, it is possible to cause a protective immunity against development of further allergies to other food allergens as well as to respiratory or contact allergens. The invention is particularly suited to protect children exhibiting a first allergic response, which are at risk to develop further allergies. The invention is particularly well suited to treat young children such as infants (0-1 year old) and toddlers (1-3 years old). Without being bound by theory, the inventors believe the reaction may be caused by a stimulation of the production of Treg cells by epicutaneous therapy, leading to a better control of IgE-producing cells and other allergy immune cells in the subject.

The invention discloses a method for preventing further allergies in a child having a detected allergic reaction, comprising epicutaneously administering to said child at least one allergen, preferably one allergen responsible for said detected allergic reaction.

The inventors have discovered that such a protective effect occurs once at least a first allergic reaction has occurred in a subject. The results presented in this application demonstrate that epicutaneous application of an allergen in such subjects can induce or stimulate Tregs. The ability to induce Tregs epicutaneously had never before been reported in the art. The results presented further show that Tregs are induced in these subjects when the allergen administered epicutaneously is an allergen responsible for the allergic reaction detected in the subject. It is therefore possible that the at least first allergic reaction (e.g., a sensitivity to an allergen) is needed to induce Treg cells production, and that the epicutaneous treatment using the allergen can amplify this Treg population thereby acting on subsequent allergic response(s). The present treatment allows generation of a non-allergen specific immune response, which inhibits the development of subsequent allergies to new allergens.

The invention is therefore particularly suited to treat children, particularly young children below the age of 6. In such children, the immune system has not been exposed yet to multiple allergens. In particular, food allergens are still limited in such children. Accordingly, upon detection of a first allergic reaction, the preventive epicutaneous treatment of the invention is particularly efficient. Moreover, because allergies can develop early, by treating these children it is possible to prevent development of multiple allergies.

The treatment is therefore preferably applied to children under 6 exhibiting an allergic response. The invention is particularly well suited for treating infants and toddlers. The allergic response detected in said children is typically a milk allergy or a dust mite allergy. Accordingly, the method is particularly suited for treating children under 6 having an allergy to milk or dust mite. By administering epicutaneously to these subjects a prophylactic amount of a milk or house dust mite allergen, it possible to effectively protect the children against development of further allergies.

It is preferred to perform the treatment shortly after detection or verification of the presence of an allergic reaction in the subject. Indeed, by acting early (i.e., before the subject has been exposed to multiple allergens), it is possible to induce a strong and potent immune response which is expected to prevent development of a wide array of allergies. It is in this regard most preferred to treat the children upon detection or verification of a first allergic reaction, so that a protective effect against all other allergens can be contemplated.

In one preferred embodiment, the allergen is administered prior to detection of a second allergic reaction to a second allergen in the treated child. Detection or verification of an allergic reaction can be performed using techniques per se known in the art. Examples of methods suitable include the use of a prick-test, the dosage of IgE, and or an atopy patch-test. The detection of an allergic reaction includes the detection of a sensitivity to an allergen, even if no clinical sign of the allergy are present. Generally, the detection comes first from appearance of classical allergic symptoms (inflammation, swallowing, etc). It may then be tested again, or verified, e.g., by a practitioner, using any of the above techniques, if needed. Once an allergic reaction has been detected or verified for a subject, treatment of the invention may start. The treatment efficacy will increase if the treatment is started shortly (e.g., immediately or within weeks, for example less than 4 weeks) after detection or verification of the allergic response. Indeed, by acting early, a preventive effect towards any other allergy is generated. For that purpose, it is best if the treatment is performed upon detection of the first allergic response in the subject, and prior to detection of a further allergy (e.g., prior to appearance of detectable IgE specific for a second allergen in said child). However, treatment is also potent in subjects showing several allergic reactions, as it can still generate to general protection against additional allergies.

The method is therefore particularly effective for treating infants (below 1 year) or toddlers (1 year to 3 years) having a first allergic reaction.

In particular, the method is for treating children under 6 having a detected allergic reaction to milk or house dust mite.

The invention also discloses a method comprising epicutaneous application of at least one allergen to the subject. Most preferably, the at least one allergen applied is an allergen responsible for the detected allergic reaction in the subject. Indeed, using such an allergen, a protective effect is obtained and the subject may also be desensitized to the allergen. Furthermore, the inventors have surprisingly shown that the causative allergen is able to induce Tregs in the subject, while unrelated allergens cannot.

An allergen "responsible for" the detected allergic reaction (or a "causative" allergen) designates any allergen derived from the substance to which the subject is allergic. The allergen may be a component of the allergic substance (e.g., a protein of milk or house dust mite), or a fragment of such a component (e.g. a fragment of a protein) having immunogenic epitope(s). As indicated before, the allergen may be a whole extract, a purified mixture, an isolated immunogen, etc.

In one embodiment of the invention, the detected allergic reaction is a milk allergy and the allergen administered comprises a milk protein or a fragment thereof having immunogenic epitope(s).

The invention also discloses that the detected allergic reaction is a house dust mite allergy and the allergen administered comprises a house dust mite protein or a fragment thereof having immunogenic epitope(s).

The epicutaneous treatment protocol may be adjusted by the practitioner. Typically, the allergen is applied repeatedly (i.e., several times), for a period of time sufficient to cause a protective immune response. In a preferred embodiment, the treatment is performed during a period of at least 3 months, preferably at least 6 months, and more preferably between 6 and 36 months. During this treatment period, the allergen may be administered at various frequencies, such as weekly, every other day, or daily. The dose of allergen used for each application can be adjusted by the skilled artisan. Typically, it may comprise between 0.1 and 10000 µg, preferably between 20 and 1000 µg. The protective immunity in the subject can be verified at any time by conventional examination. In particular, the existence of a protective immune response in the treated subjects can be verified by a decrease in, or a disappearance or absence of clinical signs of allergies, such as digestive disorders, cutaneous signs (e.g., eczema or dermatic atopy), rhinitis, or asthma. Dosing immune cells or mediators (such as IgE) in the subject may also be performed, although the absence of clinical signs is sufficient. Once the protective immunity has been generated, it is possible to reduce the treatment (dosage, frequency of application, time of application), or to even stop it. As indicated above, the treatment is typically to be maintained for a period of time of at least 3 months, with repeated applications at different intervals.

In a preferred embodiment, the allergen is applied without adjuvant. However, although not preferred, the allergen may be combined with an adjuvant, i.e., any substance that e.g., activates or accelerates the immune system to cause an enhanced immune response. Examples of adjuvants include mineral salts, such as calcium phosphate, aluminium phosphate and aluminium hydroxide; immunostimulatory DNA or RNA, such as CpG oligonucleotides; proteins, such as antibodies or Toll-like receptor binding proteins; saponins e.g. QS21; cytokines; muramyl dipeptide derivatives; LPS; MPL and derivatives including 3D-MPL; GM-CSF (Granulocyte-macrophage colony-stimulating factor); imiquimod; colloidal particles; complete or incomplete Freund's adjuvant; Ribi's adjuvant or bacterial toxin e.g. cholera toxin or enterotoxin (LT).

In a further preferred embodiment, the allergen is applied on an intact area of the skin.

It is particularly preferred to apply the allergen without adjuvant and on intact skin.

The allergen may be applied using different techniques or devices suitable to maintain contact between the allergen and the skin of the subject. Such devices include, without limitation, a patch, a tape, a dressing, a sheet, or any other form known to those skilled in the art. Preferably, the skin device is a patch, even more preferably an occlusive patch. Preferred patch devices do not alter integrity of the skin, i.e., they are non-perforant. In the most preferred embodiment, the method of the invention uses a skin patch device as described in international patent applications WO2002/071950 and WO 2007/122226. Such a device is occlusive and is configured to use an allergen in dry form, the allergen being maintained on the patch through electrostatic and/or Van der Waals forces, with no added adhesive. The preparation and characteristics of such a device (termed Viaskin®) are disclosed in detail in the above identified applications, which are incorporated herein by reference in their entireties.

For the performance of the present invention, it is particularly well suited to use a device comprising a backing adapted to create with the skin a hermetically closed chamber, this backing having on its skin facing side within the chamber the dry antigen adhered through electrostatic forces and/or Van der Waals forces. Upon application to the skin, moisture increases in the chamber, leading to allergen dissolution and contacting with the skin.

In another preferred embodiment of the invention, the antigen is applied on the skin of the subject using an occlusive patch device comprising a support to which the allergen is bound. Preferably, the allergen is bound to the support of the patch through electrostatic or Van der Waals forces, with no added adhesive. In particular embodiments, the support of the patch may comprise glass or a polymer chosen from the group consisting of cellulose plastics (CA, CP), polyvinyl chloride (PVC), polypropylenes, polystyrenes, polyurethanes, polycarbonates, polyacrylics, polyolefines, polyesters, polyethylenes and ethylene-vinyl acrylates (EVA). The patch may be secured to the skin by an adhesive border.

In a most preferred embodiment, the invention uses a dry allergen preparation, which is preferably applied on the skin using an electrostatic skin device. In this regard, the term "dry" designates the fact that the allergen is substantially powdered, e.g., in the form of particles which may be individualized or agglomerated.

Although less preferred, the allergen may be in liquid form and applied using known devices, such as occlusive devices having a reservoir or a perforated membrane.

The invention discloses a method for preventing allergy in a child, the method comprising epicutaneously administering, to a child under 6 having a detected allergic reaction to milk or house dust mite, at least one milk or house dust mite protein, said epicutaneous administration preventing the onset of allergy to other allergens in said child.

The invention also discloses a method for protecting a child from development of multiple allergies, the method comprising detecting or verifying in a child the occurrence of a first allergic reaction and, upon detection of said first allergic reaction, epicutaneously administering to said child one allergen responsible for said first allergic reaction, said epicutaneous administration inducing an immune response in said child protecting the child from development of multiple allergies.

The invention also discloses a composition for use to prevent allergy in a subject, wherein the composition comprises an allergen and is applied epicutaneously to a child having a detected allergic reaction. More preferably, the child is under 6 years old. Even more preferably, the child is allergic to milk or house dust mite. Most preferably, the child is treated upon detection of his first allergy.

The invention also discloses a method for inducing Tregs in an allergic subject, the method comprising epicutaneously applying to the subject a causative allergen.

Further aspects and advantages of the invention will be disclosed in the following experimental section, which should be considered as illustrative.

### EXAMPLES

### Methods

### Mice

Three-week-old female BALB/c mice (Charles River Laboratories, France) were used in the study. All experiments were performed according to European Community rules of animal care.

### Protocol of sensitization to milk

Three-week-old BALB/c mice were sensitized to milk by receiving 6 weekly intra-gastric administrations of milk mixed with 10µg of cholera toxin as adjuvant. Sera were collected from the retro-orbital venous plexus before and at the end of the sensitization period. Naive mice were bled on the same days.

### Protocol of epicutaneous immunotherapy to milk

Epicutaneous immunotherapy (EPIT) was performed once a week during 8 consecutive weeks. First, mice were anaesthetized intraperitoneally with ketamine and xylazine and shaved with an electric clipper and depilatory cream. The day after, 100µg of milk proteins were placed on the back and maintained by an adhesive bandage for 48 hours.

### Protocol of sensitization to peanuts

Two weeks after the end of epicutaneous immunotherapy, mice were submitted to a validated peanut sensitization program by administering only one dose (80 mg) of peanut proteins by oral route. Control mice (not sensitized to milk, not treated to milk) were submitted to the same peanut sensitization program as a positive control. Naive mice were included as negative controls.

### Sustained peanut oral challenge

Peanuts were reintroduced into their regimen. This consisted of exclusive feeding with peanuts instead of standard mouse food for 4 consecutive days. Animals then received peanuts mixed into standard food for the 6 following days, and daily intra-gastric administration of a solution containing 10 mg of peanut protein for the last 3 days of this second phase. At the end, mice were sacrificed after deep anesthesia and samples were taken to examine the histology of the esophagus, the mRNA expression of cytokines in the esophagal mucosa and the cytokines secreted by reactivated splenocytes. Blood samples were also taken in order to measure the serological responses to peanuts.

### Assay of specific IgE, IgG1, and IgG2a in plasma samples

Blood samples were collected under anaesthesia from the retro-orbital venous plexus and the plasma samples were stored at -20°C until further analyses. Specific antibodies were determined using a quantitative ELISA, which has been in-house developed. Briefly, microtiter plates were coated with 100 µl per well of milk or peanut allergenic materials at a protein concentration of 5 µg/ml. Serial dilutions of 50 µl of each serum were dispensed per well and incubated for 24 h at +4°C. An anti-mouse IgE, IgG1 or IgG2a antibody labelled with alkaline phosphatase (Serotec, Oxford, England) was used as a tracer. Para-nitrophenyl phosphate (pNPP - Sigma, France) was used as an enzyme substrate. Specific IgE, IgG1 and IgG2a were quantified by comparison with concentration-response curves obtained with a total IgE, IgG1 or IgG2a assay performed under identical conditions using a solid phase coated with an anti-mouse IgE, IgG1 or IgG2a antibody (Serotec, Oxford, England), which is complementary to tracer, instead of allergenic proteins. Mouse immunoglobulin standards were obtained from Serotec (Oxford, England).

### Cellular responses

After sacrifice of mice, spleens were teased into a single-cell suspension and washed three times in RPMI-1640 (Gibco, France). Cells were counted and adjusted to a culture density of 4 x 10⁶ cells/ml and cell suspensions of 500 µl were placed into each well of a 24-well microtiter plate (Nunc) together with 500 µl medium or milk or PPE (100µg/ml). After 72 hrs, the supernatants were harvested and analyzed for the presence of cytokines using BioPlex cytokine Assay (BioRad, marnes-la-Coquette, France) in accordance with the manufacturer's instructions. Cell count and CD4, CD25 and Foxp3 staining were performed using specific antibodies (BD Pharmingen). Acquisition was performed on Canto II cytometer by acquiring 5000 events in a predefined FSC^{lo}/SSC^{lo} gate. Percentage of CD25 and Foxp3-positive cells within CD4+ population was then assessed using FlowJo software.

### Results

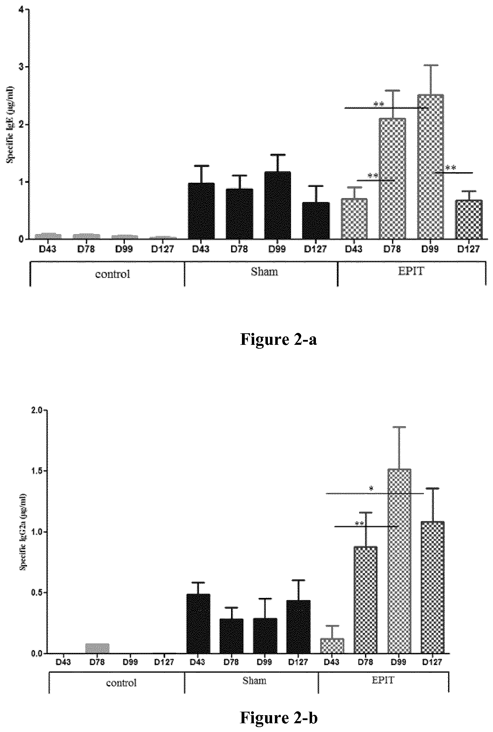

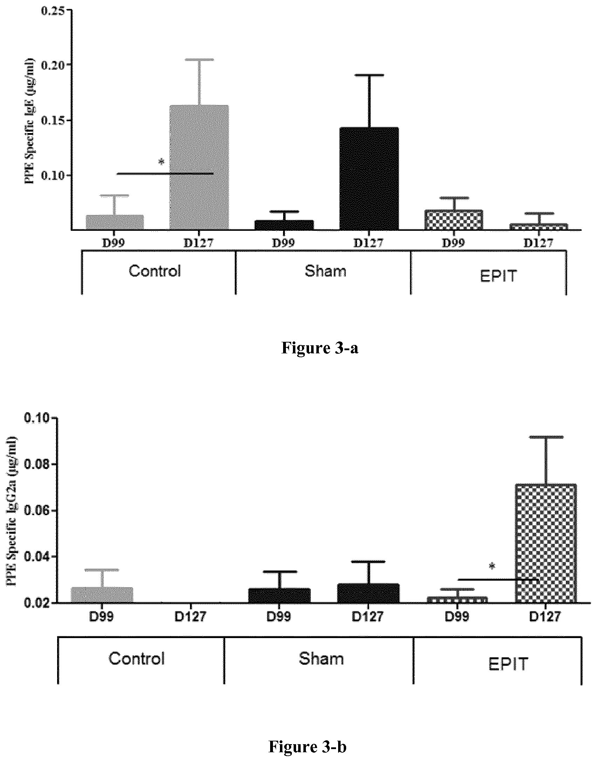

### Serological responses (figures 2 and 3)

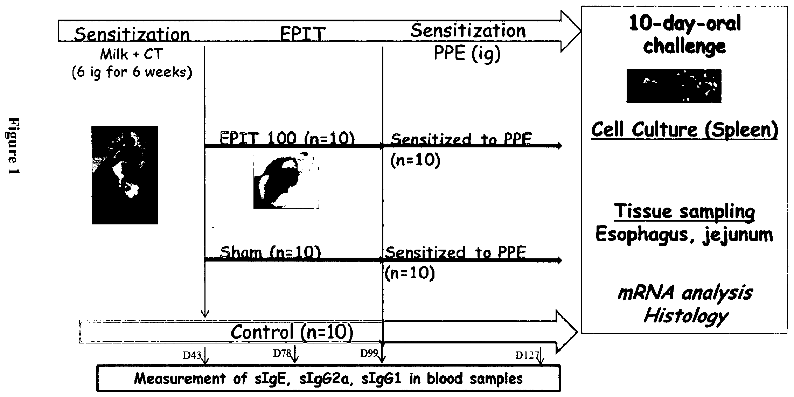

Sensitization to milk was performed from day 1 to day 43, and followed by treatment by milk EPIT (see protocol of Fig 1).

As shown in Fig 2a, sensitization to milk is marked in mice by a production of specific IgE (fig 2a: day 43).
In milk-sensitized mice, the milk EPIT treatment (fig 2a: from day 44 to day 127) induced:
- First, an increase of specific IgE (fig 2a: from day 78 to day 98) during the 8-week-period of treatment,
- then a decrease of the specific IgE (fig 2a: from day 99 to day 127) one month after the end of EPIT, as observed in clinical studies;
- eventually, a significant increase (fig 2b: from D78 to D127) of specific IgG2a, a Th1 biological marker. At day 127, the achieved rate of specific IgG2a over specific IgE showed a modification of the immune response form a Th2 profile to a more marked Th1 profile, thus signing the EPIT efficacy.

During the second phase of the protocol, mice were exposed to PPE. The results show that, among the mice exposed to peanut at day 99 (protocol of sensitization to peanut) only sham treated and control mice exhibit an increase in specific IgE to peanuts (figure 3a). To the opposite, in mice previously treated by epicutaneous milk immunotherapy, a significant increase in specific IgG2a to peanuts (figure 3b) was observed, which is characteristic of a tolerant response to peanuts. These results show that milk EPIT treatment protected the mice from subsequent PPE allergy.

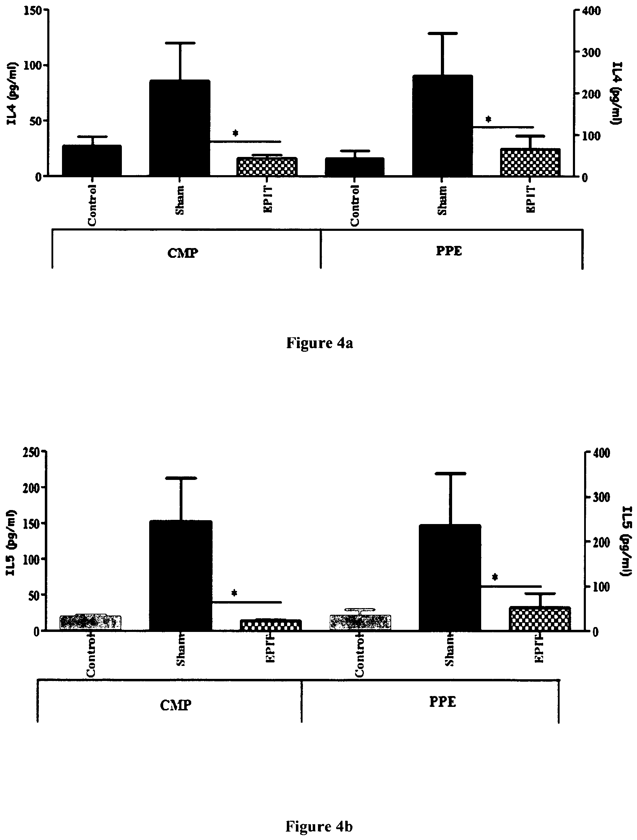

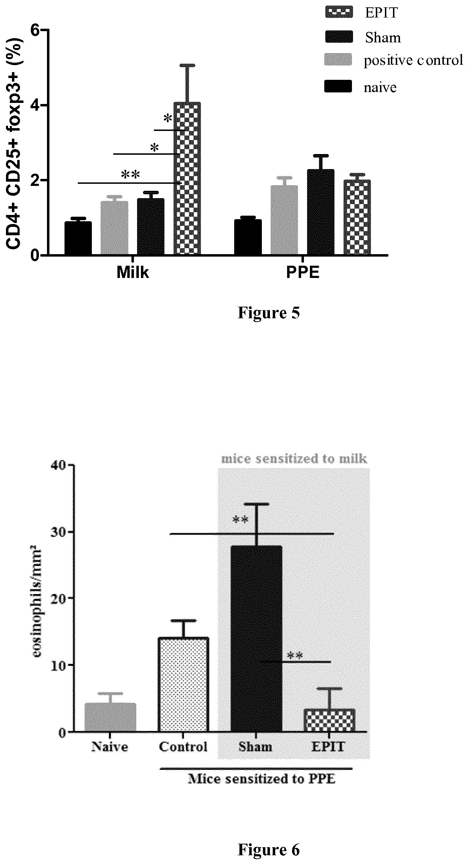

### Cellular response (figures 4 and 5)

### i) Cytokines (figure 4)

Figures 4 shows cellular responses of splenocytes reactivated in vitro by cow's milk protein (CMP) or by peanut protein extract (PPE) as measured by IL-4 (figure 4a) and IL-5 (figure 4b). Results are expressed as mean ± SD.

Figure 4-left part and figure 4b-left part: The *in vitro* reactivation of splenocytes of sham group was marked by a production of IL-4 and IL-5, which are Th2 cytokines, compared to control and EPIT (p<0.05). This phenomenon signs a protection effect of the EPIT applied to milk-EPIT mice.

Figure 5-right part and figure 5b-right part (effect of the peanut protein exposition protocol on the EPIT/sham/control groups): exposition to peanut in milk-sensitized-Sham-treated and control mice was marked by a high production of IL-4 and IL-5, which are Th2 cytokines, compared to milk sensitized-EPIT treated mice (p<0.05). This phenomenon signs a global protection effect of the EPIT applied to milk protein sensitized mice.

### ii) Treg (figure 5)

Specific induction of Treg cells was analyzed after:
- Sensitization (figure 5-milk - left part),
- Milk EPIT (figure 5-milk/ - right part),
- PPE exposure on Sham mice (figure 5 - PPE - left part),
- PPE exposure on milk EPIT treated mice (figure 5 - PPE - right part).

Our results show that, in the milk EPIT treated group, a high level of Treg (CD4⁺CD25⁺Foxp3⁺) cells is induced after milk sensitization. This Treg population is specifically induced by immunotherapy. Conversely, the number of Treg cells did not increase by *in vitro* reactivation in the presence of PPE. Without being bound by theory it seems likely that the presence of Treg cells specifically induced by EPIT-milk (figure 5-milk/ - right part), directs the immune response to PPE towards a tolerant profile without production of Th2 cytokines.

### Eosinophil infiltration in esophagus (figure 6)

The sustained oral peanut exposure to peanuts mainly triggers the esophagus and induces a high infiltration of eosinophils for mice sensitized to peanuts (control, figure 6).

A higher infiltration of eosinophils was observed in mice sensitized and not treated to milk and then exposed to peanuts with the protocol of PPE sensitization (sham, figure 6).

When mice sensitized and EPIT-treated to milk were exposed to peanuts to induce esophagus inflammation, no eosinophilic infiltration was observed in the esophagus (EPIT, figure 6), at a similar level than naive mice (mice not sensitized to milk and peanuts : naive, figure 6).

### Discussion

This study has surprisingly shown in vivo that an early desensitization to milk significantly influences the immune response to other allergens. This is the first demonstration of a preventive effect on allergy caused by epicutaneous therapy. Indeed, EPIT treatment to milk induced a specific production of regulatory T cells (Treg). The induction of tolerance to milk via Treg prevented the sensitization to other allergens, such as peanuts or HDM. It was shown that after an effective desensitization process to milk by EPIT, the treated animals were protected from sensitization to PPE and a tolerant response was obtained with a high production of specific IgG2a and low levels of Th2 cytokines secreted by reactivated splenocytes. Moreover, the protocol of desensitization to peanut induced a very low eosinophilic infiltration in esophagus.

These results therefore demonstrate that EPIT treatment can induce an effective protection against further allergies in subjects.

## Claims

1. A composition comprising a milk allergen for use in treating a child under 6 years old having a detected allergy to milk, to prevent said child from developing a further allergy to a different food allergen, wherein the composition is repeatedly administered epicutaneously to said child using a non-invasive occlusive patch device, said administration protecting the child from development of at least one further allergy to the different food allergen.

2. The composition for use of claim 1, wherein the child is an infant or a toddler.

3. The composition for use of claim 1 or 2, wherein the detected allergy is the first detected allergy in said child and wherein the allergen is administered upon detection or verification of said first allergic reaction.

4. The composition for use of any one of claims 1 to 3, wherein the allergen is applied in the absence of adjuvant.

5. The composition for use of any one of claims 1 to 4, wherein the allergen is applied on an intact area of the skin.

6. The composition for use of any one of claims 1 to 5, wherein the allergen is applied repeatedly during a period of 3 to 36 months.

7. The composition for use of any one of claims 1 to 6, in protecting said child from development of multiple allergies.

8. The composition for use of any one of claims 1 to 6, in protecting said child from development of an allergy to peanut.

## Patentansprüche

1. Eine Zusammensetzung umfassend ein Milchallergen zur Verwendung bei der Behandlung eines unter 6 Jahre alten Kindes mit nachgewiesener Allergie gegen Milch, um zu verhindern, dass das Kind eine weitere Allergie gegen ein anderes Lebensmittelallergen ausbildet, wobei die Zusammensetzung dem Kind wiederholt epikutan mittels einer nichtinvasiven Okklusivpflastervorrichtung verabreicht wird, und wobei die Verabreichung das Kind vor der Ausbildung mindestens einer weiteren Allergie gegen das andere Lebensmittelallergen schützt.

2. Die Zusammensetzung zur Verwendung nach Anspruch 1, wobei das Kind ein Baby oder ein Kleinkind ist.

3. Die Zusammensetzung zur Verwendung nach Anspruch 1 oder 2, wobei die nachgewiesene Allergie die erste nachgewiesene Allergie in dem Kind ist und wobei das Allergen bei Nachweis oder Bestätigung der ersten allergischen Reaktion verabreicht wird.

4. Die Zusammensetzung zur Verwendung nach einem der Ansprüche 1 bis 3, wobei das Allergen ohne Adjuvans angewendet wird.

5. Die Zusammensetzung zur Verwendung nach einem der Ansprüche 1 bis 4, wobei das Allergen auf einen intakten Bereich der Haut angewendet wird.

6. Die Zusammensetzung zur Verwendung nach einem der Ansprüche 1 bis 5, wobei das Allergen wiederholt über einen Zeitraum von 3 bis 36 Monaten angewendet wird.

7. Die Zusammensetzung zur Verwendung nach einem der Ansprüche 1 bis 6, zum Schutz des Kindes vor der Ausbildung multipler Allergien.

8. Die Zusammensetzung zur Verwendung nach einem der Ansprüche 1 bis 6, zum Schutz des Kindes vor der Ausbildung einer Allergie gegen Erdnüsse.

## Revendications

1. Composition comprenant un allergène de lait pour son utilisation dans le traitement d'un enfant âgé de moins de 6 ans ayant une allergie détectée au lait, pour empêcher ledit enfant de développer une autre allergie à un allergène alimentaire différent, ladite composition étant administrée de façon répétée par voie épicutanée au dit enfant en utilisant un dispositif patch occlusif non-invasif, ladite administration protégeant l'enfant contre le développement d'au moins une autre allergie à l'allergène alimentaire différent.

2. Composition pour son utilisation selon la revendication 1, où l'enfant est un nourrisson ou un enfant en bas âge.

3. Composition pour son utilisation selon la revendication 1 ou 2, où l'allergie détectée est la première allergie détectée chez ledit enfant et où l'allergène est administré dès la détection ou la vérification de ladite première réaction allergique.

4. Composition pour son utilisation selon l'une quelconque des revendications 1 à 3, où l'allergène est appliqué en l'absence d'adjuvant.

5. Composition pour son utilisation selon l'une quelconque des revendications 1 à 4, où l'allergène est appliqué sur une zone intacte de la peau.

6. Composition pour son utilisation selon l'une quelconque des revendications 1 à 5, où l'allergène est appliqué de façon répétée pendant une période de 3 à 36 mois.

7. Composition pour son utilisation selon l'une quelconque des revendications 1 à 6, pour la protection dudit enfant contre le développement d'allergies multiples.

8. Composition pour son utilisation selon l'une quelconque des revendications 1 à 6, pour la protection dudit enfant contre le développement d'une allergie à l'arachide.
